# EUROPEAN PATENT APPLICATION

(11) **EP 1 514 569 A1**
(43) Date of publication of application: **16.03.2005**
(21) Application number: 03425583.6
(22) Date of filing: 09.09.2003
(51) Int. Cl.: A61M 16/16

(54) **Bubbling humidifier of a gas, in particular oxygen, used in a respiratory therapy**

(71) Applicant: Flow Meter S.p.A., 24040 Levate (Bergamo) (IT)
(72) Inventor: Paratico, Roberto, 24040 Levate (IT)
(74) Representative: Ferreccio, Rinaldo

(57) **Abstract**

The present invention relates to a bubbling humidifier (10) of a gas, in particular oxygen, used in a respiratory therapy, which can advantageously be oriented in any position; such humidifier (10) is of the type wherein a closed container (11), which can be partially filled with water, is fixed with a substantially vertical axis (X-X) to a reducing valve (50) mounted on a cylinder (60) of said gas, said container (11) being provided with an input opening (17) for said gas coming from said reducing valve (50) and with an output opening ( 18) for humidified gas intended for an user, said input opening (17) being in fluid communication with one end (19) of a flexible and ballasted dip-tube (20), said end (19) being fixed inside the container (11), near an upper end of the container (11), an opposite free end (21) of said dip-tube (20) moving downwards because of gravity and thus being near a lower end of the container (11), when said container (11) is positioned with a substantially vertical axis (X-X).

## Description

### Field of application

The present invention relates, in its more general aspect, to a bubbling humidifier of a gas.

In particular, this invention concerns a bubbling humidifier of oxygen used in respiratory therapy where a patient is given said oxygen which is contained in a cylinder or is supplied by means of a hospital distribution system.

### Prior Art

The oxygen, coming from a cylinder or distribution system, must be humidified to a pre-established value before being given to the patient.

Bubbling humidifiers which comprise a container partially filled with water are known: the oxygen is sent from a pressure reducing valve mounted on the cylinder or from a supply system such as a flow meter by means of a flexible tube into a bottom of this container where it is made bubble. At its output, the humidifier comprises a flexible tube equipped with a respiratory mask, nasal cannula or similar for administration of the mixture to the patient.

To ensure correct operation of this type of humidifier, the container must always be placed in a predefined orientation: generally it is placed with vertical axis, hanging from a special pole or from a wall, or directly connected to the distribution device.

For practical purposes, the humidifier has also been fixed directly to the reducing valve mounted on the cylinder, thus eliminating the inconvenience of having to hang the container in a suitable position and, in this way, protecting it from being hit.

However, in this case, care must be taken so that the cylinder has always the same pre-established orientation and, more precisely, the cylinder must always operate in a vertical position.

Even if this latter type of bubbling humidifier has advantages from various points of view, it also has known drawbacks which are particularly evident when it is necessary to work in emergency conditions with the cylinder not placed in the pre-established orientation.

For example, when the cylinder is placed under a stretcher or a hospital bed: in these cases the cylinder is generally in a lying position, i.e. with substantially horizontal axis. Another significant example is when the cylinder is contained in mobile emergency units or first aid cases, which must thus have the possibility to be handled freely.

The problem underlying this invention is to devise a bubbling humidifier of a gas, in particular oxygen, used in a respiratory therapy, capable of overcoming, simply and economically, all the above-mentioned drawbacks connected to the known art.

### Summary of the invention

This problem is solved, according to the present invention, by a bubbling humidifier of a gas, in particular oxygen, used in a respiratory therapy, of the type wherein a closed container, which can be filled at least partially with water, is fixed with substantially vertically axis to a reducing valve mounted on a cylinder of said gas, said container being provided with an input opening for said gas coming from said reducing valve and an output opening for humidified gas intended for an user, characterised in that said input opening is in fluid communication with one end of a flexible and ballasted dip-tube, said end being fixed inside and near an upper end of the container, an opposite free end of said dip-tube moving because of gravity downwards and being near a lower end of the container, when said container is positioned with substantially vertical axis.

The advantages and characteristics of the bubbling humidifier of a gas, in particular oxygen, used in respiratory therapy, according to the present invention, will become clearer from the description of an embodiment thereof, made hereinafter with reference to the attached drawings given for indicative and not limiting purposes.

### Brief description of the drawings

Figure 1 schematically shows a perspective view of a bubbling humidifier of a gas, in particular oxygen, used in a respiratory therapy, according to the invention.
Figure 2 schematically shows an exploded view of the humidifier illustrated in figure 1.
Figures 3, 4 and 5 schematically show the humidifier illustrated in figure 1 in three different orientations.

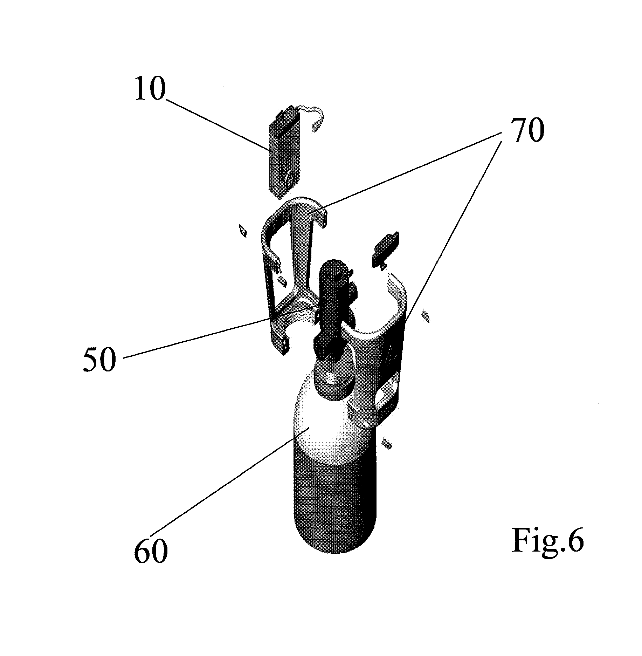

Figure 6 schematically shows a perspective view of the humidifier illustrated in figure 1 mounted on a gas cylinder.

### Detailed description of a preferred embodiment

With reference to the figures, a bubbling humidifier of a gas, in particular oxygen, used in a respiratory therapy, according to the invention, is illustrated and generally indicated with 10.

The humidifier 10 comprises a container 11 for water, having a substantially parallelepiped shape with a X-X axis. The humidifier 10 is fixed, with the X-X axis substantially vertical, to a reducing valve 50 mounted on a cylinder 60 of gas, in particular oxygen.

The container 11 is closed, at two axial ends, by an upper end 13 and a lower end14. Inside the container 11, near each of said ends 13 and 14, a membrane 12 is placed, impermeable to water but permeable to said gas, for example a membrane in porous expanded PTFE, of the type commercially known as GORE-TEX®, or a similar water repellent micro-porous fabric, which forms two watertight compartments 12a with the respective ends 13 and 14. The two watertight compartments 12a, upper and lower, are connected to each other, for example by means of a vertical chamber 15 inside the container 11, near one lateral side of the parallelepiped of the container 11.

The upper end 13 is provided with an input opening 17 for said gas and, on an upper part, with an output opening 18 for humidified gas intended for the user. The input opening 17 is fed by the reducing valve 50 and is in fluid communication with one end 19 of a dip-tube 20, said end 19 being fixed inside the container 11, near an upper end of the container 11, for example below the upper membrane 12, said membrane 12 being provided with an opening 17a to connect the dip-tube 20 with the input opening 17.

The opposite end 21 of said dip-tube 20 is free. The dip-tube 20 is flexible and ballasted in such a way that the free end 21 moves downwards because of gravity and is near a lower end of the container, when said container 11 is positioned with the axis X-X substantially vertical: for example, the opposite end 21 is positioned only slightly above the lower membrane 12. In practice, the dip-tube 20 is a flexible tube and, at the opposite end 21, it is provided with a fractionation device 16, i.e. a filter in sintered material. Alternatively, the fractionation device 16 is a diffusing element.

The dip-tube 20 is ballasted in the sense that the free end 21 tends to move downwards and generally this occurs thanks to its high flexibility and to the weight of the flexible tube and, if provided for, of the fractionation device 16. Alternatively, if this weight is not enough, the free end 21 is properly made heavier in correspondence of the fractionation device 16.

Preferably, the end 19 of the flexible tube 20 is placed out of line with respect to the axis X-X of the container 11.

Furthermore, between the two watertight compartments 12a, the container 11 is provided with an opening 22, closed by means of a removable plug, to fill the container 11 itself with water.

The humidifier 10 can be integrated with the reducing valve 50 in an open cap 70 for the cylinder 60, thus forming a complete unit for oxygen-therapy.

The operation of the bubbling humidifier of a gas, in particular oxygen, used in respiratory therapy, according to the invention, is now given in more detail.

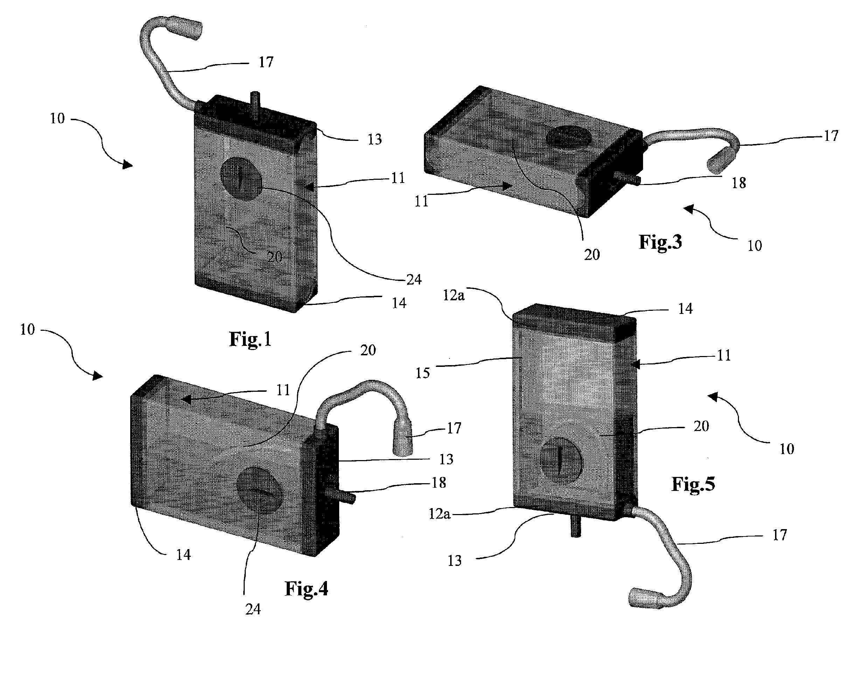

Reference is first made to figure 1, where the humidifier 10 has the axis X-X positioned vertically.

When the reducing valve 50 supplies the gas, it reaches the flexible tube 20 and comes out at the opposite free end 21. This end 21 is immersed in water since the container 11 has been previously filled at least partially with water through the opening 22 then closed with the plug 24.

The gas rises towards the water surface, bubbling, and is thus humidified by the pressure of the water itself. filters The humidified gas crosses the upper membrane 12, the upper membrane 12 retaining only the residual humidity. In this way the humidified gas reaches the upper watertight compartment 12a, wherefrom it exits through the output opening 18 towards the patient in respiratory therapy by means of a flexible tube with mask, nasal cannula or similar.

In this case, the cylinder 60 has axis positioned vertically. If the cylinder were positioned in a lying position, i.e. with horizontal axis, the humidifier 10 would continue to operate correctly, as illustrated in figures 3 and 4, where the parallelepiped of the container 11 is positioned with different lateral sides lying horizontally, respectively.

In fact, a portion of the membrane 12, upper and/or lower, is free from water (since the container 11 is generally filled only partially with water) and is, therefore, permeable to the mixture of oxygen enriched with humidity. The opposite end 21 of the flexible tube 20 remains immersed in water since it bends as a consequence of gravity towards an area of the container 11 where the contained water is collected.

Even in the case where the cylinder 60 is turned upside-down, the humidifier 10 of the invention continues to operate correctly.

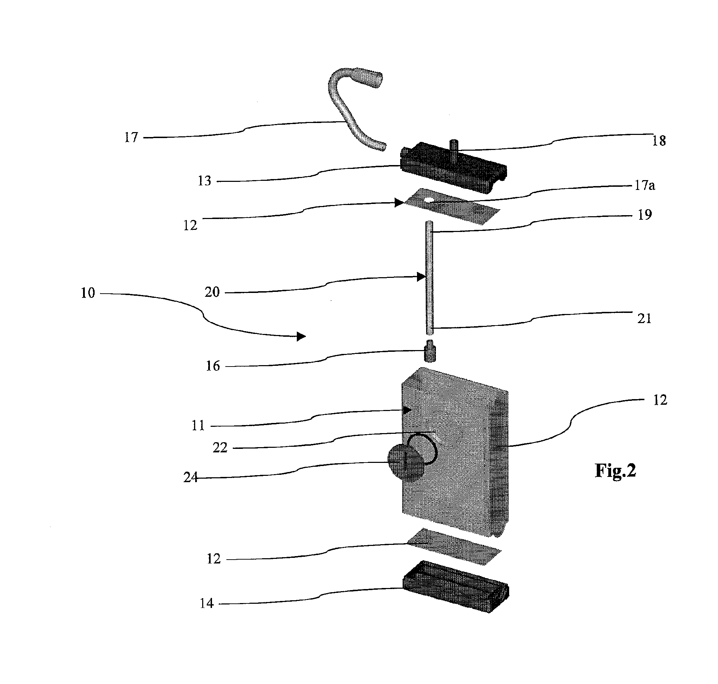

In this case, with reference to figure 5, the flexible tube 20 bends over itself as a consequence of gravity, substantially forming an upside-down "U": in practice, a siphon is generated which prevents water from returning into the input opening 17. This characteristic is of fundamental importance since it prevents water from running out towards the reducing valve 50, thus damaging it.

In this upside-down position it is pointed out that, in case of a possible obstruction of the output opening 18, the membrane 12, completely covered by water, cannot be damaged since the watertight compartment 12a which is below the membrane 12 is connected to the other watertight compartment 12a by means of the vertical chamber 15: in fact, in this way, the balancing of the internal pressures in a humidified gas collection chamber is guaranteed, this chamber being given by the two watertight compartments 21 a together with the vertical chamber 15.

It is further pointed out how the membranes 12 make it possible to retain the water inside the container 11, so making the humidifier outwards watertight, even when rotating its position.

Furthermore, it should be noted how the membranes 12 in micro-porous fabric, for example porous expanded PTFE of the type known as GORE-TEX®, guarantee antibacterial and antiviral protection: this protects the patient from cross infections deriving from formations which can develop in a humid environment, such as that of the container 11.

The main advantage achieved by the bubbling humidifier of a gas, in particular oxygen, used in respiratory therapy, of the present invention is that it can be used in any position, thus making it unusually practical its use.

It is also pointed out how the humidifier of the invention can be advantageously integrated with the reducing valve in an open cylinder cap, thus making it possible to obtain a complete system for oxygen therapy.

The bubbling humidifier of a gas, in particular oxygen, used in respiratory therapy, as described above is susceptible to modifications all within the reach of a skilled in the art and all covered by the scope of protection of the invention as defined in the following claims.

## Claims

1. A bubbling humidifier (10) of a gas, in particular oxygen, used in a respiratory therapy, of the type wherein a closed container (11), which can be filled at least partially with water, is fixed with a substantially vertical axis (X-X) to a reducing valve (50) mounted on a cylinder (60) of said gas, said container (11) being provided with an input opening (17) for said gas coming from said reducing valve (50) and an output opening (18) for humidified gas intended for an user, **characterised in that** said input opening (17) is in fluid communication with one end (19) of a flexible and ballasted dip-tube (20), said end (19) being fixed inside the container (11), near an upper end of the container (11), an opposite free end (21) of said dip-tube (20) moving downwards because of gravity and being near a lower end of the container (11), when said container (11) is positioned with a substantially vertical axis (X-X).

2. Humidifier (10) according to claim 1, **characterised in that** said container (11) has a substantially parallelepiped shape and is closed, at two axial ends, by an upper end (13) and by a lower end (14).

3. Humidifier (10) according to claim 2, **characterised in that**, inside said container (11), near each of said ends (13, 14), a membrane (12) is placed, impermeable to said liquid and permeable to said gas, which forms two watertight compartments (12a) with said respective end s (13, 14), said two watertight compartments (12a), upper and lower, being connected to each other.

4. Humidifier (10) according to claim 3, **characterised in that** said connection is obtained by means of a vertical chamber (15) placed inside said container (11), near one lateral side of the parallelepiped of the container (11).

5. Humidifier (10) according to claim 3, **characterised in that** said membrane (12) is in water repellent micro-porous GORE-TEX® or similar fabric.

6. Humidifier (10) according to claim 3, **characterised in that** said upper end (13) is provided with said input opening (17) and, on top, with said output opening (18), said end (19) of said dip-tube (20) being fixed below the upper membrane (12), said membrane (12) being provided with an opening (17a) to connect said dip-tube (20) to said input opening (17).

7. Humidifier (10) according to claim 6, **characterised in that** said opposite end (21) of said dip-tube (20) is located slightly above said lower membrane (12).

8. Humidifier (10) according to claim 1, **characterised in that** said dip-tube (20) is a flexible tube.

9. Humidifier (10) according to claim 1, **characterised in that** said dip-tube (20) is provided, at said opposite end (21), with a fractionation device (16).

10. Humidifier (10) according to claim 9, **characterised in that** said fractionation device (16) is a filter in sintered material or a diffusing element.

11. Humidifier (10) according to claim 1, **characterised in that** said end (19) of said dip-tube (20) is placed out of line with respect to the axis (X-X) of the container (11).

12. Humidifier (10) according to claim 3, **characterised in that** said container (11), between said two watertight compartments (12a), is provided with an opening (22), closed by a removable plug (24).

13. Humidifier (10) according to claim 1, **characterised in that** it is integrated with said reducing valve (50) in an open cap (70) for said cylinder (60).
